# EUROPEAN PATENT SPECIFICATION

(11) **EP 4 392 118 B1**
(45) Date of publication and mention of the grant of the patent: **18.03.2026**
(21) Application number: 21787179.7
(22) Date of filing: 10.09.2021
(51) Int. Cl.: A61M 39/02

(54) **SIDE ACCESS FOR DUAL LUMEN PORTS**
SEITENZUGANG FÜR DOPPELLUMEN-PORTS
ACCÈS LATÉRAL POUR ORIFICES À DOUBLE LUMIÈRE

(43) Date of publication of application: 03.07.2024
(73) Proprietor: Bard Peripheral Vascular, Inc., Franklin Lakes, NJ 07417 (US)
(72) Inventor: ANDERSEN, Christian, Kaysville, UT 84037 (US); FIUMEFREDDO, Diana, Salt Lake City, UT 84121 (US); THOMAS, Ian N., Bountiful, UT 84087 (US); HOYE, Jessica, Phoenix, AZ 85032 (US); DENSLEY, Bryon Ray, Rochester, MN 55902 (US)
(74) Representative: Hoffmann Eitle
(86) International application number: PCT/US2021/049922
(87) International publication number: WO 2023/038634

(56) References cited:
- US-A- 5 562 618
- US-A1- 2005 075 614
- US-A1- 2011 098 663

## Description

### BACKGROUND

US 5 562 618 A discloses a portal assembly including a port with at least one resealable septum, and a tube extending from the port with the tube being sized to be received inside a catheter, an angled surface surrounding the tube wherein the angled surface diverges away from the tube in a direction away from the port, and sleeve structure for forcing the end of the catheter into the angled surface, thereby forcing the end of the catheter radially inwardly toward the tube and forming a thickened portion adjacent the end of the catheter to seal and hold the catheter. In a dual port construction, the outlet tube are parallel to one another at their distal ends, and the sleeve structure forces the end of the catheter into the angled surface.

US 2011/098663 A1 discloses an implantable port including a body with a reservoir in the body, a tunnel extending from the reservoir to an outer surface of the body, a pivoting coupling connected to the body near the tunnel on the outer surface, and a stem connected to the pivoting coupling.

US 2005/075614 A1 discloses a chamber for the infusion of a medicament, the chamber comprising a reservoir, an outer casing having a base wall and a lateral wall, a region for access to the reservoir, and a duct for the diffusion of the medicament.

### SUMMARY

Briefly summarized, embodiments disclosed herein are directed to dual, in-line ports having a side access stem. Dual, in-line ports include two or more reservoirs aligned along a longitudinal axis, where the longitudinal axis extends parallel to an axis of subcutaneous insertion. Advantageously, dual, in-line ports require a small incision site, relative to side-by-side dual port designs, i.e. ports where two or more reservoirs are aligned at an angle, e.g. perpendicular, to the longitudinal axis. Typically port stems extend from a side of the port that is proximate the incision site, when the port is disposed subcutaneously. However, for in-line ports, a fluid path between the catheter and the reservoir that is furthest from the stem can be tortuous. This can extend the fluid path and can impact fluid flow and pressures, relative to a fluid path from the reservoir adjacent the stem, leading to inconsistencies between the two fluid pathways.

A first aspect relates to a subcutaneously implantable access port according to claim 1.

In some embodiments, the central port axis extends horizontally through a diametric center point of the first reservoir and through a diametric center point of the second reservoir. In some embodiments, the first stem axis extends perpendicular to the central port axis. In some embodiments, the second stem axis extends parallel to the central port axis. In some embodiments, the base extends from the body between the diametric center point of the first reservoir and the diametric center point of the second reservoir.

In some embodiments, the body extends along the central port axis between a first end and a second end, the first reservoir disposed proximate the first end, and the second reservoir disposed proximate the second end, and wherein the base of the stem extends from the body between a first edge of the second reservoir disposed proximate the first end, a second edge of the first reservoir disposed proximate the second end.

A second aspect relates to a subcutaneously implantable access port system according to claim 8.

In some embodiments, the second stem axis can be angled relative to the first stem axis between a first position and a second position. In some embodiments, the second stem axis can be angled relative to the first stem axis between 0°-90°. In some embodiments, the second stem axis can be angled relative to the first stem axis along a horizontal plane, a vertical plane, or along a plane extending at an angle therebetween. In some embodiments, the subcutaneously implanted access port further includes a channel disposed in an outer surface of the body and configured to receive a portion of the catheter therein.

Also disclosed is an unclaimed method of placing a catheter and port system subcutaneously including, forming an incision site, coupling the catheter with a stem of the port, the stem defining a first stem lumen and a second stem lumen, and inserting the port through the incision site along a central port axis, the port including a first reservoir and a second reservoir disposed along the central port axis, and a stem having a base extending from the port along a first stem axis between the first reservoir and the second reservoir, and a nozzle extending from the base along a second stem axis angled relative to the first stem axis.

In some examples, the method further includes inserting a first end of the port through the incision site before the second end of the port, disposed opposite the first end, the first end disposed proximate the first reservoir and the second end disposed proximate the second reservoir. In some examples, the method further includes the first stem lumen providing fluid communication between the first reservoir and a first catheter lumen, and the second stem lumen providing fluid communication between the second reservoir and a second catheter lumen. In some examples, the first stem axis extends perpendicular to the central port axis, and the second stem axis extends perpendicular to first stem axis.

In some examples, the second stem axis extends parallel to the central port axis. In some examples, the method further includes pivoting the second stem axis of the nozzle between a first position and a second position relative to the first stem axis of the base. In some examples, the method further includes pivoting the second stem axis of the nozzle relative to the first stem axis of the base through one of a horizontal plane, vertical plane, or along a plane extending at an angle therebetween. In some examples, the method further includes pivoting the second stem axis of the nozzle relative to the first stem axis of the base through an angle of between 0° and 90°. In some examples, the method further includes securing a portion of the catheter within a channel defined within a surface of the port body.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a perspective view of a dual, in-line port, catheter, and an angled side stem, in accordance with embodiments disclosed herein.
FIG. 1B shows a horizontal cross-section view of a dual, in-line port, catheter, and an angled side stem, in accordance with embodiments disclosed herein.
FIG. 1C shows a vertical, lateral cross-section view of a dual, in-line port having a channel, in accordance with embodiments disclosed herein.
FIG. 2A shows a plan view of a dual, in-line port having pivotable stem, in accordance with embodiments disclosed herein.
FIG. 2B shows a stem side view of a dual, in-line port having pivotable stem, in accordance with embodiments disclosed herein.
FIG. 2C shows a second end view of a dual, in-line port having pivotable stem, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

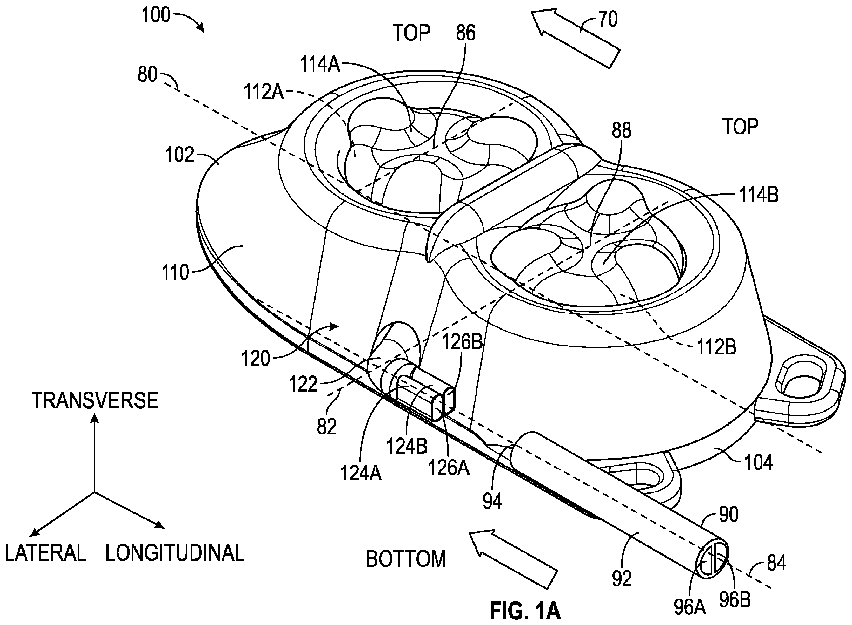

To assist in the description of embodiments described herein, as shown in FIGS. 1A-1B, a longitudinal axis extends substantially parallel to an axis of subcutaneous insertion 70. A lateral axis extends normal to the longitudinal axis, and a transverse axis extends normal to both the longitudinal and lateral axes. As used herein, a horizontal plane extends along the lateral and longitudinal axes. A vertical plane extends normal to the horizontal plane.

Unless defined otherwise, all technical and scientific terms used herein have the same meaning as commonly understood by those of ordinary skill in the art.

FIGS. 1A-1B show an embodiment of a subcutaneous vascular access device system, or "port" 100. In an embodiment, the port 100 includes a body 110 defining one or more reservoirs 112. The body 110 extends along a longitudinal axis between a first end 102 and a second end 104. As shown in FIGS. 1A-1B, the port 100 is a "dual, in-line" port 100 and includes two or more reservoirs, including a first reservoir 112A, and a second reservoir 112B. The first reservoir 112A and the second reservoir 112B are arranged along a longitudinal axis such that a central port axis 80 extends parallel to the longitudinal axis, and may extend through a diametric center point 86 of the first reservoir 112A and a diametric center point 88 of the second reservoir 112B.

In an embodiment, the reservoir 112 can include a first edge 132 disposed proximate the first end 102 of the port 100 and a second edge 134 disposed proximate the second edge 104 of the port 100. For example the first reservoir 112A can include a first edge 132A and a second edge 134A disposed opposite the first edge 132A across the diametric center point 86 of the first reservoir 112A. The second reservoir 112B can include a first edge 132B and a second edge 134B disposed opposite the first edge 132B across the diametric center point 88 of the second reservoir 112B.

In use, the port 100 can be placed subcutaneously in a first direction 70, through an incision site, with a first end 102 passing through the incision site before the second end 104. As such, the first reservoir 112A and the second reservoir 112B can be "in-line" with an axis of subcutaneously placement extending parallel to the longitudinal axis.

The port 100 can further include one or more septa 114 disposed over the reservoir 112 and configured to provide percutaneous access thereto. For example, a first septum 114A can be disposed over the first reservoir 112A, and a second septum 114B can be disposed over the second reservoir 112B. It will be appreciated that other configurations of septum 114 and reservoir 112 are also contemplated. For example, a first septum 114A can be disposed over both the first reservoir 112A and the second reservoir 112B. These and other configurations of septum 114 and reservoir 112 are contemplated to fall within the scope of the present invention. In an embodiment, an access needle can extend percutaneously, through the needle-penetrable septum 114 and into the reservoir 112 to provide fluid communication therewith.

The port 100 further includes a stem 120 extending from a surface of the port body 110. The stem 120 includes a base 122 extending from the port body 110 along a first stem axis 82, and a nozzle 124 extending along a second stem axis 84. The first stem axis 82 extends at an angle relative to the central port axis 80. In an embodiment, the first stem axis 82 can extend at an angle of between 1° and 180° relative to the central port axis 80. In an embodiment, the first stem axis 82 can extend at an angle of between 45° and 135° relative to the central port axis 80. In an embodiment, the first stem axis 82 can extend perpendicular relative to the central port axis 80.

The second stem axis 84 extends at an angle relative to the first stem axis 82. In an embodiment, the second stem axis 84 can extend at an angle of between 1° and 180° relative to the first stem axis 82. In an embodiment, the second stem axis 84 can extend at an angle of between 45° and 135° relative to the first stem axis 82. In an embodiment, the second stem axis 82 can extend perpendicular relative to first stem axis 82. In an embodiment, the second stem axis 82 can extend parallel to the central port axis 80.

In an embodiment, the base 122 of the stem 120 can extend from the port body 110 at a point disposed longitudinally between the first end 102 and the second end 104 of the port body 110. In an embodiment, the base 122 of the stem 120 can extend from the port body 110 at a point disposed longitudinally between the diametric center point 86 of the first reservoir 112A and the diametric center point 88 of the second reservoir 112B. In an embodiment, the base 122 of the stem 120 can extend from the port body 110 at a point disposed longitudinally between the second end 134A of the first reservoir 112A and a first end 132A of the second reservoir 112B. In an embodiment, the base 122 of the stem 120 can extend from the port body 110 at a mid-point disposed longitudinally between the diametric center point 86 of the first reservoir 112A and the diametric center point 88 of the second reservoir 112B.

The stem 120 defines a first stem lumen 126A in fluid communication with the first reservoir 112A, and a second stem lumen 126B in fluid communication with the second reservoir 112B. In an embodiment, the stem nozzle 124 can include a first stem nozzle 124A defining a portion of the first stem lumen 126A, and a second stem nozzle 124B defining a portion of the second stem lumen 126B. The first stem nozzle 124A can engage a first lumen 96A and the second stem nozzle 124B can engage a second lumen 96B, in and interference fit, press-fit, or snap-fit engagement, or combinations thereof.

In an embodiment, the catheter 90 can include a catheter body 92 defining one or more catheter lumen 96, e.g. the first catheter lumen 96A and the second catheter lumen 96B, and extending between a distal tip and a proximal end 94. In an embodiment, the proximal end 94 of the catheter 90 can be coupled to the stem nozzle 124 by urging the catheter axially thereover. In an embodiment, the proximal end 94 of the catheter 90 can be trimmable prior to coupling the catheter 90 with the stem 80. In an embodiment, the catheter 90 can be formed of a flexible or compliant material configured to elastically deform radially outwards and engage the stem nozzle 124 in an interference fit. In an embodiment, the catheter 90 can be formed of a plastic, polymer, elastomer, rubber, silicone rubber, combinations thereof, or the like.

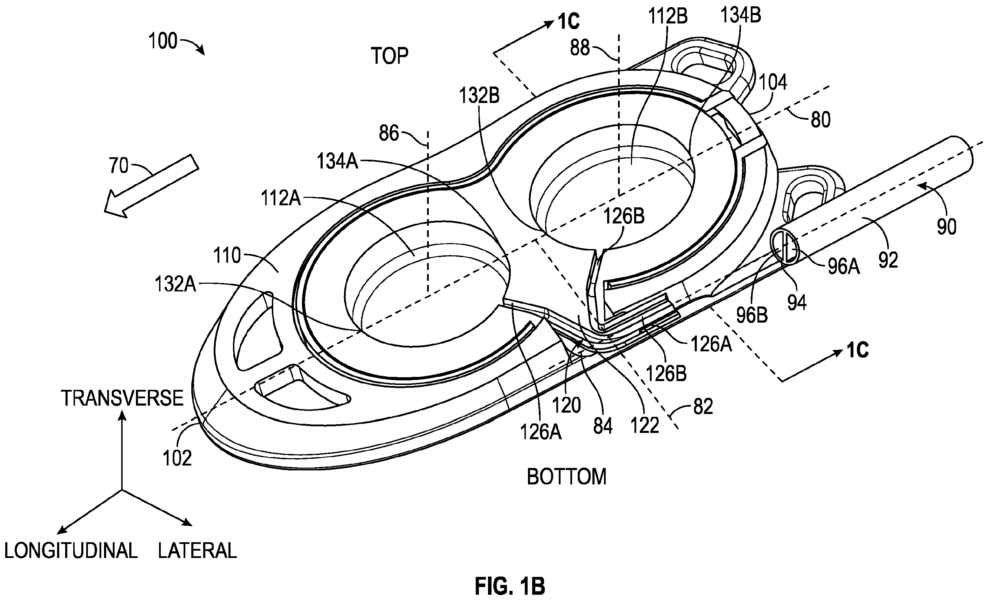

As shown in FIGS. 1B, with the stem base 122 extending from a side of the port body 110 at a longitudinal mid-point between the diametric center point 86 of the first reservoir 112A and the diametric center point 88 of the second reservoir 112B, the first stem lumen 126A and the second stem lumen 126B can define a similar distance. For example, a distance between the diametric center point 86 of the first reservoir 112A and the tip of the first nozzle 124A can be substantially the same as a distance between the diametric center point 88 of the second reservoir 112B and the tip of the second nozzle 124B.

In an embodiment, the first stem lumen 126A and the second stem lumen 126B can define a similar degree of tortuousness. For example, a number of inflexion points of the first stem lumen 126A between the diametric center point 86 of the first reservoir 112A and the tip of the first nozzle 124A can be substantially the same as a number of inflexion points of the second stem lumen 126B between the diametric center point 88 of the second reservoir 112B and the tip of the second nozzle 124B. As such, in an embodiment, a flow resistance of the first stem lumen 126A can be equal to a flow resistance of the second stem lumen 126B. Worded differently, a difference in flow resistance between the first stem lumen 126A and a second stem lumen 126B can be zero, or can provide a *de minimis* difference in flow resistance.

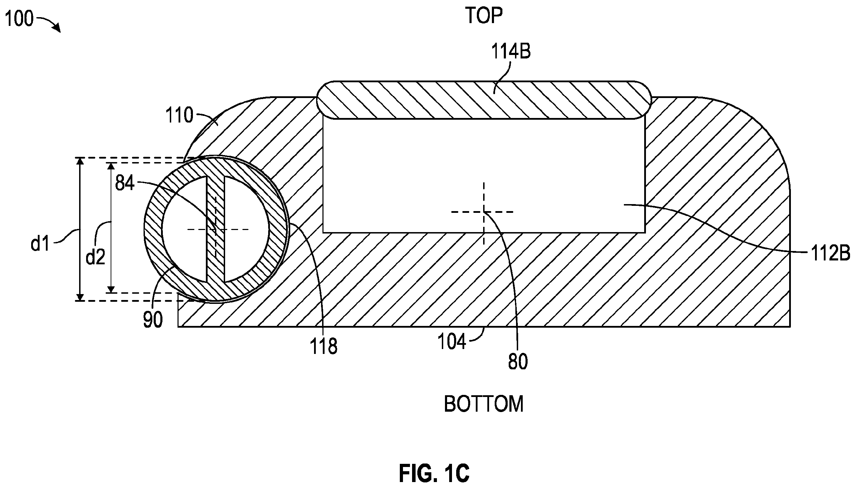

In an embodiment, the second stem axis 84 of the stem nozzle 124 can extend substantially parallel to the central port axis 80 to provide a reduced lateral profile when inserting the port 100 in a first direction 70 through an insertion site. In an embodiment, as shown in FIG. 1C, the port body 110 can define a channel 118 configured to receive a portion of the catheter 90 therein. FIG. 1C shows a cross-section view of the port 100, extending laterally through the second reservoir 112B. The channel 118 can extend parallel to a central port axis 80 and can receive the portion of the catheter 90 within a volume defined by the port body 110 to provide a substantially continuous outer profile. Further the channel 118 can provide a reduced lateral profile since an axis of the catheter, substantially at second stem axis 84 can extend within a volume defined by the port 100, rather than adjacent a volume defined by the port 100. In an embodiment, the channel 118 can retain a portion of the catheter 90 in an interference fit, press-fit, or snap-fit engagement. For example, a diameter (*d1*) of an opening to the channel 118, extending along a transverse axis, can be less than an outer diameter (*d2*) of the catheter 90. As such, the channel 118 can retain and further stabilize a portion of the catheter 90 that extends along a surface of the port body 110. In an embodiment, the port 100 can include one or more clips or similar structures configured to retain and stabilize a portion of the catheter 90 extending from the stem nozzle 124.

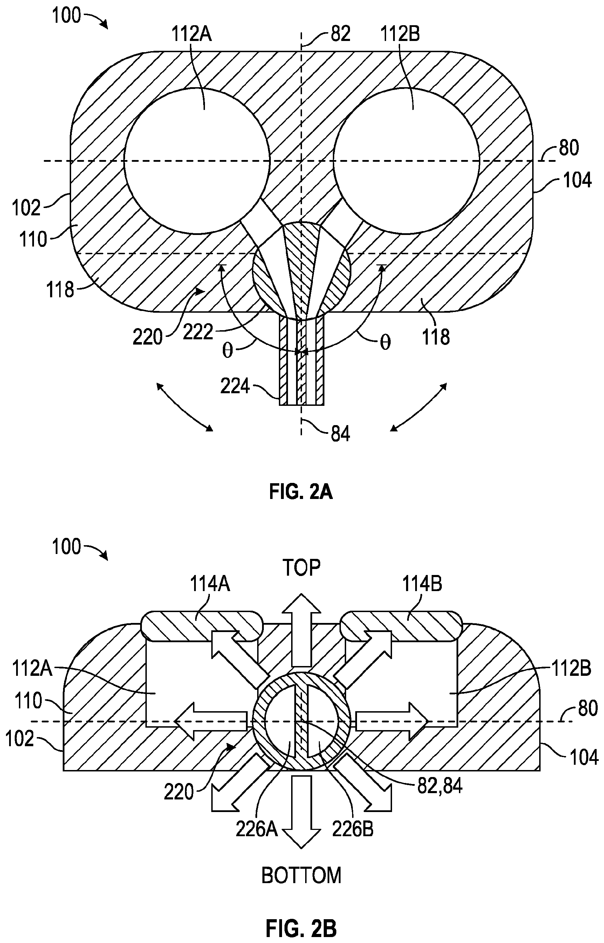

As shown in FIGS. 2A-2C, in an embodiment, the port 100 includes a pivotable stem 220 having a base 222 extending from the port body 110 along a first stem axis 82. The stem further includes a nozzle 224 extending from the base 222 and defining a second stem axis 84. The base 222 is pivotably coupled to the port body 110 such that the nozzle 224 extending along the second stem axis 84 can pivot relative to the first stem axis 82. In an embodiment, the base 222 can include a hinge, a ball-and-socket joint, flexible portion, or similar mechanism, configured to allow the stem 220 to pivot relative to the port body 110 while the stem lumen 226A, 226B can maintain fluid communication between the catheter 90 and the reservoirs 112A, 112B. In an embodiment, the second stem axis 84 can pivot through a horizontal plane, a vertical plane, or through a plane angled relative thereto. In an embodiment, the second stem axis 84 can pivot through an angle (*Θ*) of between 0° and 90° relative to the first stem axis 82. As noted, the stem lumen 226 can maintain fluid communication at each direction and at each angle (*Θ*).

For example, FIG. 2A shows a plan view of the port 100, the nozzle 224 defining the second stem axis 84 can pivot through an angle (*Θ*) through a horizontal plane. As such the second stem axis 84 of the nozzle 224 can be directed towards either the first end 102, towards the second end 104, i.e. can be aligned parallel to the central port axis 80, or can be positioned at an angle relative to the central port axis 80.

FIG. 2B shows a stem side view of the port 100, the nozzle 224 defining the second stem axis 84 can pivot through an angle (*Θ*) through a horizontal plane, vertical plane, or along a plane angled relative thereto. As such the second stem axis 84 can angled relative to the first stem axis 84 and the nozzle 224 can be directed towards either the first end 102, towards the second end 104, towards to top side, towards a bottom side, or can be positioned at an angle therebetween.

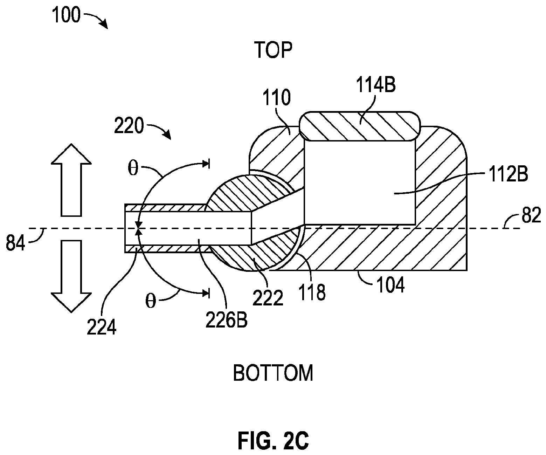

FIG. 2C shows a second end, cross-section view of the port 100, the nozzle 224 defining the second stem axis 84 can pivot through an angle (*Θ*) through a vertical plane, or along a plane angled relative thereto. As such the second stem axis 84 can be angled relative to first stem axis 82 and the nozzle 224 can be directed towards either the top side, towards the bottom side, or can be positioned at an angle therebetween. As used herein, a top side of the port 100 can be disposed proximate a skin surface, when the port 100 is placed subcutaneously.

In an exemplary unclaimed method of use, a port 100 can be provided including a pivotable stem 220, as described herein. A catheter 90 can be coupled to the stem nozzle 224 by urging the nozzle 224 into a lumen of the catheter 90. For example, a first nozzle 124A can engage a first lumen 96A and a second nozzle 124B can engage a second lumen 96B. The nozzle 224 can engage the lumen 96 in an interference fit, as described herein. The second stem axis 84 of the nozzle and thereby an axis of the catheter 90 coupled thereto can be then angled relative to the first stem axis 82. For example, the catheter 90 can be positioned along a side surface of the port 100 to minimize a lateral cross-sectional profile of the port 100 and catheter assembly 90. The port 100 and catheter assembly 90 can then be placed subcutaneously along the longitudinal port axis 80, with a first end 102 passing through the incision site before the second end 104.

Advantageously, the pivotable stem 220 can allow a user to reposition the axis of the catheter 90 (i.e. the second stem axis 84) relative to the port 100 between a first position and a second position, to facilitate subcutaneous placement. The stem lumen 226 can maintain fluid communication between the reservoir 112 and the catheter lumen 96 at each of the first position and the second position. The port 100 can require a smaller incision site relative to a side-by-side port design as well as provide improved fluid pathways for both first stem lumen 226A and the second stem lumen 226B, providing a similar fluid pathway distance, similar degree of tortuous pathway, i.e. similar inflexion points, and providing a similar fluid resistance through either of the first stem lumen 226A and the second stem lumen 226B.

While some particular embodiments have been disclosed herein, and while the particular embodiments have been disclosed in some detail, it is not the intention for the particular embodiments to limit the scope of the concepts provided herein. Accordingly, departures may be made from the particular embodiments disclosed herein without departing from the scope of the concepts provided herein.

## Claims

1. A subcutaneously implantable dual, in-line access port (100), comprising:
a body (110) defining a first reservoir (112A) and a second reservoir (112B) aligned along a longitudinal axis of the body such that a central port axis (80) extends parallel to the longitudinal axis and between the first reservoir (112A) and the second reservoir (112B);
a stem (120, 220) having a base (122, 222) extending from the body (110) along a first stem axis (82) angled relative to the central port axis (80), and a nozzle (124, 224) extending from the base (122, 222) along a second stem axis (84) and angled relative to the first stem axis (82), the base (122, 222) extending from the body (110) between the first reservoir (112A) and the second reservoir (112B), the stem (120, 220) defining a first stem lumen (126A, 226A) in fluid communication with the first reservoir (112A) and a second stem lumen (126B, 226B) in fluid communication with the second reservoir (112B).

2. The subcutaneously implantable access port (100) according to claim 1, wherein the central port axis (80) extends horizontally through a diametric center point (86) of the first reservoir (112A) and through a diametric center point (88) of the second reservoir (112B).

3. The subcutaneously implantable access port (100) according to any one of claims 1-2, wherein the first stem axis (82) extends perpendicular to the central port axis (80).

4. The subcutaneously implantable access port (100) according to any one of claims 1-3, wherein the second stem axis (84) extends parallel to the central port axis (80).

5. The subcutaneously implantable access port (100) according to any one of claims 1-4, wherein the base (122, 222) extends from the body (110) between the diametric center point (86) of the first reservoir (112A) and the diametric center point (88) of the second reservoir (112B).

6. The subcutaneously implantable access port (100) according to any one of claims 1-5, wherein the body (110) extends along the central port axis (80) between a first end (102) and a second end (104), the first reservoir (112A) disposed proximate the first end (102), and the second reservoir (112B) disposed proximate the second end (104), and wherein the base (122, 222) of the stem (120, 220) extends from the body (110) between a first edge (132) of the second reservoir (112B) disposed proximate the first end (102), and a second edge (134) of the first reservoir (112A) disposed proximate the second end (104).

7. The subcutaneously implantable access port (100) according to any one of claims 1-6, wherein the body (110) defines a channel (118) configured to receive a portion of a catheter (90).

8. A subcutaneously implantable access port system, comprising:
a catheter (90) defining a first catheter lumen (96A) and a second catheter lumen (96B);
a port (100) having a body (110) defining a first reservoir (112A) and a second reservoir (112B), a central port axis (80) extending therebetween; and
a stem (220) having a base (222) pivotably coupled to the body (110) and extending from the body (110) along a first stem axis (82) angled relative to the central port axis (80), and a nozzle (224) extending from the base (222) along a second stem axis (84), the base (222) extending from the body (110) between the first reservoir (112A) and the second reservoir (112B), and the stem (220) defining a first stem lumen (226A) configured to provide fluid communication between with the first reservoir (112A) and the first catheter lumen (96A), and a second stem lumen (226B) configured to provide fluid communication between the second reservoir (112B) and the second catheter lumen (96B), wherein the stem (120, 220) is pivotable relative to the body (110) while maintaining fluid communication between the catheter (90) and the first and second reservoirs (112A, 112B).

9. The subcutaneously implantable access port system according to claim 8, wherein the second stem axis (84) can be angled relative to the first stem axis (82) between a first position and a second position.

10. The subcutaneously implantable access port system according to any one of claims 8-9, wherein the second stem axis (84) can be angled relative to the first stem axis (82) between 0°-90°.

11. The subcutaneously implantable access port system according to any one of claims 8-10, wherein the second stem axis (84) can be angled relative to the first stem axis (82) along a horizontal plane, a vertical plane, or along a plane extending at an angle therebetween.

12. The subcutaneously implantable access port system according to any one of claims 8-11, further including a channel (118) disposed in an outer surface of the body (110) and configured to receive a portion of the catheter (90) therein.

## Patentansprüche

1. Subkutan implantierbarer dualer Inline-Zugangsport (100), umfassend:
einen Körper (110), der ein erstes Reservoir (112A) und ein zweites Reservoir (112B) definiert, die entlang einer Längsachse des Körpers ausgerichtet sind, sodass sich eine zentrale Portachse (80) parallel zu der Längsachse und zwischen dem ersten Reservoir (112A) und dem zweiten Reservoir (112B) erstreckt;
einen Schaft (120, 220), der eine Basis (122, 222) aufweist, die sich von dem Körper (110) entlang einer ersten Schaftachse (82) erstreckt, die relativ zu der zentralen Portachse (80) angewinkelt ist, und eine Düse (124, 224), die sich von der Basis (122, 222) entlang einer zweiten Schaftachse (84) erstreckt und relativ zu der ersten Schaftachse (82) angewinkelt ist, wobei sich die Basis (122, 222) von dem Körper (110) zwischen dem ersten Reservoir (112A) und dem zweiten Reservoir (112B) erstreckt, wobei der Schaft (120, 220) ein erstes Schaftlumen (126A, 226A) definiert, das in Fluidverbindung mit dem ersten Reservoir (112A) steht, und ein zweites Schaftlumen (126B, 226B) definiert, das in Fluidverbindung mit dem zweiten Reservoir (112B) steht.

2. Subkutan implantierbarer Zugangsport (100) nach Anspruch 1, wobei sich die zentrale Portachse (80) horizontal durch einen durchmessermittigen Punkt (86) des ersten Reservoirs (112A) und durch einen durchmessermittigen Punkt (88) des zweiten Reservoirs (112B) erstreckt.

3. Subkutan implantierbarer Zugangsport (100) nach einem der Ansprüche 1-2, wobei sich die erste Schaftachse (82) senkrecht zu der zentralen Portachse (80) erstreckt.

4. Subkutan implantierbarer Zugangsport (100) nach einem der Ansprüche 1-3, wobei sich die zweite Schaftachse (84) parallel zu der zentralen Portachse (80) erstreckt.

5. Subkutan implantierbarer Zugangsport (100) nach einem der Ansprüche 1-4, wobei die Basis (122, 222) sich von dem Körper (110) zwischen dem durchmessermittigen Punkt (86) des ersten Reservoirs (112A) und dem durchmessermittigen Punkt (88) des zweiten Reservoirs (112B) erstreckt.

6. Subkutan implantierbarer Zugangsport (100) nach einem der Ansprüche 1-5, wobei sich der Körper (110) entlang der zentralen Portachse (80) zwischen einem ersten Ende (102) und einem zweiten Ende (104) erstreckt, wobei das erste Reservoir (112A) nahe dem ersten Ende (102) angeordnet ist und das zweite Reservoir (112B) nahe dem zweiten Ende (104) angeordnet ist, und wobei sich die Basis (122, 222) des Schafts (120, 220) von dem Körper (110) zwischen einer ersten Kante (132) des zweiten Reservoirs (112B), die nahe dem ersten Ende (102) angeordnet ist, und einer zweiten Kante (134) des ersten Reservoirs (112A), die nahe dem zweiten Ende (104) angeordnet ist, erstreckt.

7. Subkutan implantierbarer Zugangsport (100) nach einem der Ansprüche 1-6, wobei der Körper (110) einen Kanal (118) definiert, der zum Aufnehmen eines Abschnitts eines Katheters (90) konfiguriert ist.

8. Subkutan implantierbares Zugangsportsystem, umfassend:
einen Katheter (90), der ein erstes Katheterlumen (96A) und ein zweites Katheterlumen (96B) definiert;
einen Port (100), der einen Körper (110) aufweist, der ein erstes Reservoir (112A) und ein zweites Reservoir (112B) definiert, wobei sich eine zentrale Portachse (80) dazwischen erstreckt; und
einen Schaft (220), der eine Basis (222) aufweist, die mit dem Körper (110) schwenkbar gekoppelt ist und sich von dem Körper (110) entlang einer ersten Schaftachse (82) erstreckt, die relativ zu der zentralen Portachse (80) angewinkelt ist, und eine Düse (224), die sich von der Basis (222) entlang einer zweiten Schaftachse (84) erstreckt, wobei sich die Basis (222) von dem Körper (110) zwischen dem ersten Reservoir (112A) und dem zweiten Reservoir (112B) erstreckt, und wobei der Schaft (220) ein erstes Schaftlumen (226A) definiert, das konfiguriert ist, eine Fluidverbindung zwischen dem ersten Reservoir (112A) und dem ersten Katheterlumen (96A) bereitzustellen, und ein zweites Schaftlumen (226B) definiert, das konfiguriert ist, eine Fluidverbindung zwischen dem zweiten Reservoir (112B) und dem zweiten Katheterlumen (96B) bereitzustellen, wobei der Schaft (120, 220) relativ zu dem Körper (110) schwenkbar ist, während eine Fluidverbindung zwischen dem Katheter (90) und den ersten und zweiten Reservoirs (112A, 112B) aufrechterhalten bleibt.

9. Subkutan implantierbares Zugangsportsystem nach Anspruch 8, wobei die zweite Schaftachse (84) relativ zu der ersten Schaftachse (82) zwischen einer ersten Stellung und einer zweiten Stellung angewinkelt werden kann.

10. Subkutan implantierbares Zugangsportsystem nach einem der Ansprüche 8-9, wobei die zweite Schaftachse (84) relativ zu der ersten Schaftachse (82) zwischen 0°-90° angewinkelt werden kann.

11. Subkutan implantierbares Zugangsportsystem nach einem der Ansprüche 8-10, wobei die zweite Schaftachse (84) relativ zu der ersten Schaftachse (82) entlang einer horizontalen Ebene, einer vertikalen Ebene oder entlang einer Ebene angewinkelt werden kann, die sich unter einem Winkel zwischen diesen erstreckt.

12. Subkutan implantierbares Zugangsportsystem nach einem der Ansprüche 8-11, weiter einen Kanal (118) einschließend, der in einer Außenfläche des Körpers (110) angeordnet ist und der konfiguriert ist, um einen Abschnitt des Katheters (90) darin aufzunehmen.

## Revendications

1. Orifice d'accès implantable sous-cutané double et en ligne (100), comprenant :
un corps (110) définissant un premier réservoir (112A) et un deuxième réservoir (112B) alignés le long d'un axe longitudinal du corps de telle sorte qu'un axe central d'orifice (80) s'étend parallèlement à l'axe longitudinal et entre le premier réservoir (112A) et le deuxième réservoir (112B) ;
une tige (120, 220) présentant une base (122, 222) s'étendant depuis le corps (110) le long d'un premier axe de tige (82) incliné par rapport à l'axe central d'orifice (80), et une buse (124, 224) s'étendant depuis la base (122, 222) le long d'un deuxième axe de tige (84) et inclinée par rapport au premier axe de tige (82), la base (122, 222) s'étendant depuis le corps (110) entre le premier réservoir (112A) et le deuxième réservoir (112B), la tige (120, 220) définissant une première lumière de tige (126A, 226A) en communication fluidique avec le premier réservoir (112A) et une deuxième lumière de tige (126B, 226B) en communication fluidique avec le deuxième réservoir (112B).

2. Orifice d'accès implantable sous-cutané (100) selon la revendication 1, dans lequel l'axe central d'orifice (80) s'étend horizontalement à travers un point central diamétral (86) du premier réservoir (112A) et à travers un point central diamétral (88) du deuxième réservoir (112B).

3. Orifice d'accès implantable sous-cutané (100) selon l'une quelconque des revendications 1 à 2, dans lequel le premier axe de tige (82) s'étend perpendiculairement à l'axe central d'orifice (80).

4. Orifice d'accès implantable sous-cutané (100) selon l'une quelconque des revendications 1 à 3, dans lequel le deuxième axe de tige (84) s'étend parallèlement à l'axe central d'orifice (80).

5. Orifice d'accès implantable sous-cutané (100) selon l'une quelconque des revendications 1 à 4, dans lequel la base (122, 222) s'étend depuis le corps (110) entre le point central diamétral (86) du premier réservoir (112A) et le point central diamétral (88) du deuxième réservoir (112B).

6. Orifice d'accès implantable sous-cutané (100) selon l'une quelconque des revendications 1 à 5, dans lequel le corps (110) s'étend le long de l'axe central d'orifice (80) entre une première extrémité (102) et une seconde extrémité (104), le premier réservoir (112A) étant disposé à proximité de la première extrémité (102), et le deuxième réservoir (112B) étant disposé à proximité de la seconde extrémité (104), et dans lequel la base (122, 222) de la tige (120, 220) s'étend depuis le corps (110) entre un premier bord (132) du deuxième réservoir (112B) disposé à proximité de la première extrémité (102), et un second bord (134) du premier réservoir (112A) disposé à proximité de la seconde extrémité (104).

7. Orifice d'accès implantable sous-cutané (100) selon l'une quelconque des revendications 1 à 6, dans lequel le corps (110) définit un canal (118) configuré pour recevoir une partie d'un cathéter (90).

8. Système d'orifice d'accès implantable sous-cutané, comprenant :
un cathéter (90) définissant une première lumière de cathéter (96A) et une deuxième lumière de cathéter (96B) ;
un orifice (100) présentant un corps (110) définissant un premier réservoir (112A) et un deuxième réservoir (112B), un axe central d'orifice (80) s'étendant entre ceux-ci ; et
une tige (220) présentant une base (222) couplée de manière pivotante au corps (110) et s'étendant depuis le corps (110) le long d'un premier axe de tige (82) incliné par rapport à l'axe central d'orifice (80), et une buse (224) s'étendant depuis la base (222) le long d'un deuxième axe de tige (84), la base (222) s'étendant depuis le corps (110) entre le premier réservoir (112A) et le deuxième réservoir (112B), et la tige (220) définissant une première lumière de tige (226A) configurée pour fournir une communication fluidique entre le premier réservoir (112A) et la première lumière de cathéter (96A), et une deuxième lumière de tige (226B) configurée pour fournir une communication fluidique entre le deuxième réservoir (112B) et la deuxième lumière de cathéter (96B), dans lequel la tige (120, 220) est pivotable par rapport au corps (110) tout en maintenant une communication fluidique entre le cathéter (90) et les premier et deuxième réservoirs (112A, 112B).

9. Système d'orifice d'accès implantable sous-cutané selon la revendication 8, dans lequel le deuxième axe de tige (84) peut être incliné par rapport au premier axe de tige (82) entre une première position et une seconde position.

10. Système d'orifice d'accès implantable sous-cutané selon l'une quelconque des revendications 8 à 9, dans lequel le deuxième axe de tige (84) peut être incliné par rapport au premier axe de tige (82) entre 0°-90°.

11. Système d'orifice d'accès implantable sous-cutané selon l'une quelconque des revendications 8 à 10, dans lequel le deuxième axe de tige (84) peut être incliné par rapport au premier axe de tige (82) le long d'un plan horizontal, d'un plan vertical, ou le long d'un plan s'étendant selon un angle entre ceux-ci.

12. Système d'orifice d'accès implantable sous-cutané selon l'une quelconque des revendications 8 à 11, incluant en outre un canal (118) disposé dans une surface externe du corps (110) et configuré pour recevoir en son sein une partie du cathéter (90).
